(19) Europäisches Patentamt
European Patent Office
Office européen des brevets

(11) **EP 4 653 845 A1**

(12) **EUROPEAN PATENT APPLICATION**

(43) Date of publication:
**26.11.2025 Bulletin 2025/48**

(21) Application number: **25174893.5**

(22) Date of filing: **07.05.2025**

(51) International Patent Classification (IPC):
**G01N 21/359** *(2014.01)*  **B01F 35/213** *(2022.01)*
**B01F 35/22** *(2022.01)*  **G01N 21/84** *(2006.01)*
**G01N 21/65** *(2006.01)*

(52) Cooperative Patent Classification (CPC):
**G01N 21/359;** G01N 21/65; G01N 2021/8411;
G01N 2201/129

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.05.2024 US 202418671510**

(71) Applicant: **Viavi Solutions Inc.
Chandler, AZ 85286 (US)**

(72) Inventors:
• **SUN, Lan
Santa Rosa, CA 95404 (US)**
• **HSIUNG, ChangMeng
Redwood City, CA 94065 (US)**

(74) Representative: **Murgitroyd & Company
165-169 Scotland Street
Glasgow G5 8PL (GB)**

(54) **MULTIPLICATIVE SCATTER CORRECTION BASED ANALYSIS FOR DYNAMIC PROCESS END POINT DETECTION**

(57) Methods and devices for multiplicative scatter correction based analysis for dynamic process, such a blending process, end point detection. A method comprises: receiving, by a device (220), spectroscopic data (102) associated with an iteration of a dynamic process; generating (104), by the device (220) and based on the spectroscopic data, a set of parameter profiles associated with the iteration of the dynamic process, wherein each parameter profile in the set of parameter profiles corresponds to a respective parameter in a set of parameters of a physical signal associated with the iteration of the dynamic process; and determining (106) an end point of the iteration of the dynamic process based on the set of parameter profiles.

100 →

FIG. 1A

**Description**

BACKGROUND

A dynamic process, such as a blending process (e.g., a blending process associated with manufacturing a pharmaceutical product), may involve one or more transitions in state, such as a transition from an unsteady state (e.g., a heterogenous state of a blending at which properties of a blend vary with time) to a steady state (e.g., a homogeneous state of blending at which the properties of the blend remain substantially constant with time). For example, a blending process may involve a transition where spectral properties of a blend transition from an unsteady state (e.g., at a start of the blending process) to a steady state (e.g., indicating that the blending process is complete).

SUMMARY

In some implementations, a method includes receiving, by a device, spectroscopic data associated with an iteration of a dynamic process; generating, by the device and based on the spectroscopic data, a set of parameter profiles associated with the iteration of the dynamic process, wherein each parameter profile in the set of parameter profiles corresponds to a respective parameter in a set of parameters of a physical signal associated with the iteration of the dynamic process; and determining an end point of the iteration of the dynamic process based on the set of parameter profiles.

The set of parameter profiles may be generated using a multiplicative scatter correction (MSC) model that extracts the set of parameter profiles from the spectroscopic data.

The set of parameter profiles may include a profile of a parameter indicating an additive effect of light scattering during the iteration of the dynamic process.

The set of parameter profiles may include a profile of a parameter indicative of a multiplicative effect of light scattering during the iteration of the dynamic process.

Determining the end point of the iteration of the dynamic process may comprises: determining a set of trends, wherein each trend in the set of trends may correspond to a respective parameter profile in the set of parameter profiles; generating, a set of slope profiles, wherein each slope profile in the set of slope profiles may be associated with a respective parameter profile in the set of parameter profiles; identifying sets of slope thresholds associated with the iteration of the dynamic process based on the set of trends and the set of slope profiles; determining a set of candidate end points based on the set of slope profiles and the sets of slope thresholds, wherein each candidate end point in the set of candidate end points may be associated with a respective slope profile in the set of slope profiles; and determining the end point of the iteration of the dynamic process based on the set of candidate end points.

The end point may be a first candidate end point, and the method further may comprise: determining a second candidate end point of the iteration of the dynamic process based on the spectroscopic data, wherein the second candidate end point may be determined based on a chemical signal associated with the iteration of the dynamic process; and selecting either the first candidate end point or the second candidate end point as a final end point of the iteration of the dynamic process.

Determining the end point of the iteration of the dynamic process may comprise: identifying a set of parameter thresholds associated with the iteration of the dynamic process based on the set of parameter profiles; and determining the end point of the iteration of the dynamic process based on the set of parameter profiles and the set of parameter thresholds.

The method may further comprise identifying, based on the spectroscopic data, a starting time point of the spectroscopic data to be used for determining the end point of the iteration of the dynamic process, wherein a starting time point associated with generating the set of parameter profiles may be at or after the identified starting time point of the spectroscopic data.

In some implementations, a device includes one or more memories; and one or more processors, coupled to the one or more memories, configured to: obtain spectroscopic data associated with an iteration of a dynamic process; generate, based on the spectroscopic data, a set of parameter profiles associated with the iteration of the dynamic process, wherein the set of parameter profiles are generated using a multiplicative scatter correction (MSC) model that extracts the set of parameter profiles from the spectroscopic data; and determine an end point of the iteration of the dynamic process based on the set of parameter profiles.

Each parameter profile in the set of parameter profiles may correspond to a respective parameter in a set of parameters of a physical signal associated with the iteration of the dynamic process.

The set of parameter profiles may include a profile of a parameter indicating an additive effect of light scattering during the iteration of the dynamic process.

The set of parameter profiles may include a profile of a parameter indicative of a multiplicative effect of light scattering during the iteration of the dynamic process.

The one or more processors, to determine the end point of the iteration of the dynamic process, may be configured to: determine a set of trends, wherein each trend in the set of trends may correspond to a respective parameter profile in the set of parameter profiles; generate, a set of slope profiles, wherein each slope profile in the set of slope profiles may be associated with a respective parameter profile in the set of parameter profiles; identify sets of slope thresholds associated with the iteration of the dynamic process based on the set of trends and the set of slope profiles; determine a set of candidate end points based on the set of slope profiles and the sets of slope thresholds, wherein each candidate end point in the set of candidate end points may be associated with a respective slope profile in the set of slope profiles; and determine the end point of the iteration of the dynamic process based on the set of candidate end points.

The end point may be a first candidate end point, and the one or more processors may further be configured to: determine a second candidate end point of the iteration of the dynamic process based on the spectroscopic data, wherein the second candidate end point may be determined based on a chemical signal associated with the iteration of the dynamic process; and select either the first candidate end point or the second candidate end point as a final end point of the iteration of the dynamic process.

The one or more processors, to determine the end point of the iteration of the dynamic process, may be configured to: identify a set of parameter thresholds associated with the iteration of the dynamic process based on the set of parameter profiles; and determine the end point of the iteration of the dynamic process based on the set of parameter profiles and the set of parameter thresholds.

The one or more processors may further be configured to identify, based on the spectroscopic data, a starting time point of the spectroscopic data to be used for determining the end point of the iteration of the dynamic process, wherein a starting time point associated with generating the set of parameter profiles may be at or after the identified starting time point of the spectroscopic data.

In some implementations, a non-transitory computer-readable medium storing a set of instructions includes one or more instructions that, when executed by one or more processors of a device, cause the device to: receive spectroscopic data associated with an iteration of a dynamic process; generate, based on the spectroscopic data, a set of parameter profiles associated with the iteration of the dynamic process, wherein the set of parameter profiles includes at least one of: a profile of a parameter indicating an additive effect of light scattering during the iteration of the dynamic process, or a profile of a parameter indicative of a multiplicative effect of light scattering during the iteration of the dynamic process; and determine an end point of the iteration of the dynamic process based on the set of parameter profiles.

The set of parameter profiles may be generated using a multiplicative scatter correction (MSC) model that extracts the set of parameter profiles from the spectroscopic data.

Each parameter profile in the set of parameter profiles may correspond to a respective parameter in a set of parameters of a physical signal associated with the iteration of the dynamic process.

The one or more instructions, that cause the device to determine the end point of the iteration of the dynamic process, may cause the device to: determine a set of trends, wherein each trend in the set of trends may correspond to a respective parameter profile in the set of parameter profiles; generate, a set of slope profiles, wherein each slope profile in the set of slope profiles may be associated with a respective parameter profile in the set of parameter profiles; identify sets of slope thresholds associated with the iteration of the dynamic process based on the set of trends and the set of slope profiles; determine a set of candidate end points based on the set of slope profiles and the sets of slope thresholds, wherein each candidate end point in the set of candidate end points may be associated with a respective slope profile in the set of slope profiles; and determine the end point of the iteration of the dynamic process based on the set of candidate end points.

BRIEF DESCRIPTION OF THE DRAWINGS

Figs. 1A-1I are diagrams associated with determining an end point of iteration of a dynamic process based on a set of parameter profiles, as described herein.

Fig. 2 is a diagram of an example environment in which systems and/or methods described herein may be implemented.

Fig. 3 is a diagram of example components of a device, which may correspond to spectrometer, detection device, and/or user device.

Fig. 4 is a flowchart of an example process associated with determining an end point of iteration of a dynamic process based on a set of parameter profiles.

DETAILED DESCRIPTION

The following detailed description of example implementations refers to the accompanying drawings. The same reference numbers in different drawings may identify the same or similar elements. The following description uses a spectrometer as an example. However, the techniques, principles, procedures, and methods described herein may be used with any sensor, including but not limited to other optical sensors and spectral sensors.

As noted above, a dynamic process may involve a transition where spectral properties of a test subject transition from an unsteady state to a steady state. For example, a blending process may involve a transition where spectral properties of a blend of materials transition from an unsteady state (e.g., a state at which properties of the blend of materials vary with time) to a steady state (e.g., a state at which the properties of the blend of materials remain substantially constant with time). The steady state indicates that blending has been achieved. Therefore, accurate and reliable detection of the steady state based on spectral properties of the test subject can both improve performance of the dynamic process (e.g., by ensuring adequate blending) and increase efficiency of the dynamic process (e.g., by enabling a blending process to be ended as soon as blending has been achieved).

Conventional techniques for detecting an end point of an iteration of a blending process based on spectral properties use a moving block analysis, which may include the use of, for example, a moving block standard deviation (MBSD), a moving block mean (MBM), a moving block relative standard deviation (MB-RSD), or a moving F-test. A moving block analysis typically requires selection of thresholds for such variables (e.g., the MBSD and/or the MBM), based on calibration or historical data, to determine when a steady state is reached. However, in practice, reproducing identical iterations of a blending process is difficult to achieve, particularly during development of a blending process. This means that selection of appropriate thresholds to be used for end point detection is challenging, which can result in inaccurate end point detection.

Further, the conventional techniques for detecting an end point of an iteration of a blending process based on spectral properties are based on chemical signals of materials being blended. However, during blending, particles of the different materials being blended move around and gradually mix together. This means that a physical signal, such as a light scattering signal, would change during the iteration of the blending process (in addition to a chemical signal changing during the iteration of the blending process). Such a physical signal could therefore be used in association with detecting the end point of the blending process (e.g., as an alternative to using a chemical signal or complementary to using the chemical signal).

Some implementations described herein enable multiplicative scatter correction (MSC)-based analysis for dynamic process end point detection. That is, the techniques and apparatuses described herein enable determination of an end point of an iteration of a dynamic process (e.g., a blending process) using an MSC-based analysis. In some implementations, a device may receive spectroscopic data associated with an iteration of a dynamic process. The device may then generate, based on the spectroscopic data, a set of parameter profiles associated with the iteration of the dynamic process. Here, each parameter profile corresponds to a respective parameter in a set of parameters of a physical signal associated with the iteration of the dynamic process. The device may then determine an end point of the iteration of the dynamic process based on the set of parameter profiles.

In some implementations, the techniques and apparatuses described herein take advantage of changes with respect to a physical property (e.g., light scattering) exhibited during an iteration of a blending process, thereby enabling information related to blend homogeneity to be provided from a physical perspective (in addition to or in alternative to a chemical perspective). In some implementations, the techniques and apparatuses described herein can be used to complement a conventional analysis that uses a chemical signal, thereby improving reliability of end point detection (e.g., by enabling a detected end point to be verified or confirmed).

In some implementations, a shape-based technique can be used in association with determining an end point of an iteration of a dynamic process during post-analysis of one or more iterations using an MSC-based technique. Here, the shape-based technique does not require calibration or historical data, and can be employed without a need for a user to manually select thresholds used for end point detection. In some implementations, thresholds for end point detection can be identified automatically (e.g., without user intervention), and these thresholds can be used to monitor future iterations of the dynamic process (e.g., in real-time or near real-time). Notably, while examples described herein are described in the context of a blending process, these techniques and apparatuses can be used for any dynamic process that proceeds from an unsteady state toward a steady state. Additional details are described below.

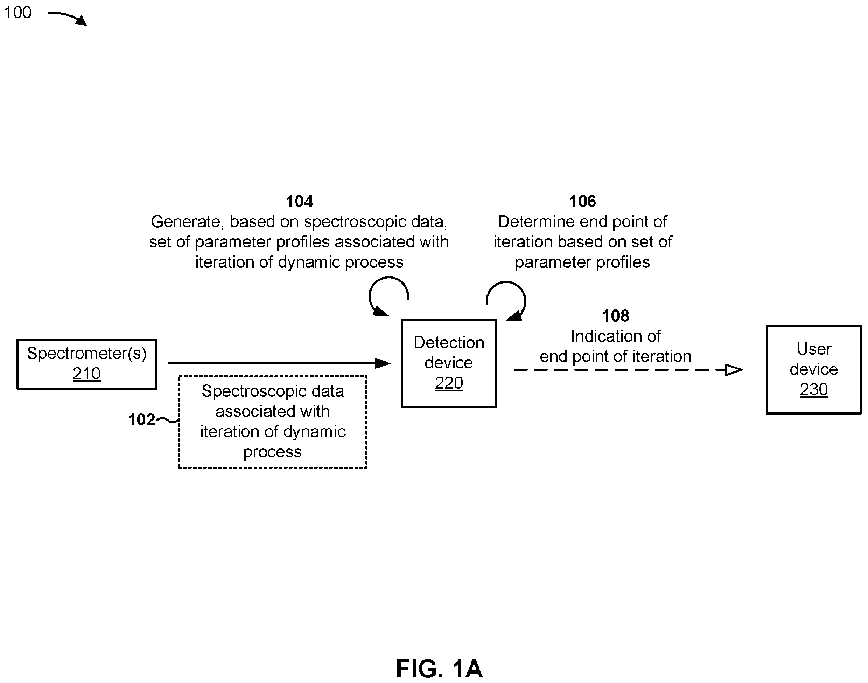

Figs. 1A-1I are diagrams associated with determining an end point of an iteration of a dynamic process based on a set of parameter profiles, as described herein. Fig. 1A is a diagram illustrating an example implementation 100 associated with determining an end point of an iteration of a dynamic process based on a set of parameter profiles. As shown in Fig. 1A, the example implementation 100 includes a spectrometer 210, a detection device 220, and a user device 230.

As shown in Fig. 1A at reference 102, the detection device 220 may receive spectroscopic data associated with an iteration of a dynamic process. For example, as shown, the spectrometer 210 may measure spectroscopic data at a given time during the iteration of the blending process, and may provide the spectroscopic data to the detection device 220. In some implementations, the spectroscopic data includes spectra (e.g., multivariate time series data, such as near-infrared (NIR) spectra) measured by the spectrometer 210 during the iteration of the blending process.

In some implementations, the detection device 220 may receive the spectroscopic data in real-time or near real-time during the iteration of the blending process. For example, detection device 220 may receive spectroscopic data, measured by the spectrometer 210 during the iteration of the blending process, in real-time or near real-time relative to the spectrometer 210 obtaining the spectroscopic data during the blending process. In some implementations, the detection device 220 may, based on the spectroscopic data, determine an end point of the iteration of the dynamic process, as described herein.

In some implementations, the detection device 220 may preprocess the spectroscopic data. For example, the raw spectroscopic data may include some amount of noise, a scattering effect, an artifact, or other type of unwanted feature. Therefore, in some implementations, the detection device 220 may preprocess the spectroscopic data to reduce a presence of or remove such unwanted features from the spectroscopic data. In some implementations, the detection device 220 may preprocess the spectroscopic data using, for example, a derivative calculation technique, a standard normal variate (SNV) technique, or an MSC technique, among other examples.

As shown at reference 104, the detection device 220 may generate a set of parameter profiles associated with the iteration of the dynamic process based on the spectroscopic data. In some implementations, the detection device 220 may generate the set of parameter profiles using an MSC model that is configured to extract the set of parameter profiles from the spectroscopic data. For example, the spectroscopic data may include a time-series of NIR spectra collected during an iteration of a blending process. As noted above, a conventional analysis utilizes chemical information extracted from the spectroscopic data to monitor the iteration of the blending process and determine the end point (e.g., a point in time at which blend homogeneity is reached). However, baselines of the time series of spectra change following a pattern that can be attributed to light scattering of materials during blending. This is understandable because, during blending, particles of different materials move and gradually mix together, which results in changes of light scattering. Thus, the iteration of the blending process can be monitored by monitoring changes of a physical signal representative of light scattering throughout the iteration of the blending process. In some implementations, the MSC model may be configured to model an additive effect of light scattering (referred to as an alpha ($\alpha$) parameter), a multiplicative effect of light scattering (referred to as a beta ($\beta$) parameter), and/or another type of parameter associated with light scattering. In one example, an NIR spectrum $x_i$ can be theoretically described by Equation 1:

$$x_i = \alpha_i + \beta_i x_{i,chem} + e_i \qquad (1)$$

where $\alpha_i$ represents an additive baseline shift, $\beta_i$ represents a multiplicative effect, $x_{i,chem}$ is a theoretical spectrum without any noise or scattering effect, and $e_i$ represents an error vector describing random measurement noise and other scattering effects not explicitly described by Equation 1. Notably, while $\alpha$ and $\beta$ parameters are shown in Equation 1 and are used in examples described herein, an extended MSC (EMSC) model or another type of model including additional or different parameters can be used in a similar fashion.

In some implementations, each parameter profile in the set of parameter profiles corresponds to a respective parameter in a set of parameters of a physical signal associated with the iteration of the dynamic process. For example, with respect to Equation 1, the set of parameter profiles may include a profile of a parameter indicating an additive effect of light scattering during the iteration of the dynamic process (i.e., an $\alpha$ profile). As another example, the set of parameter profiles may include a profile of a parameter indicative of a multiplicative effect of light scattering during the iteration of the dynamic process (i.e., a $\beta$ profile).

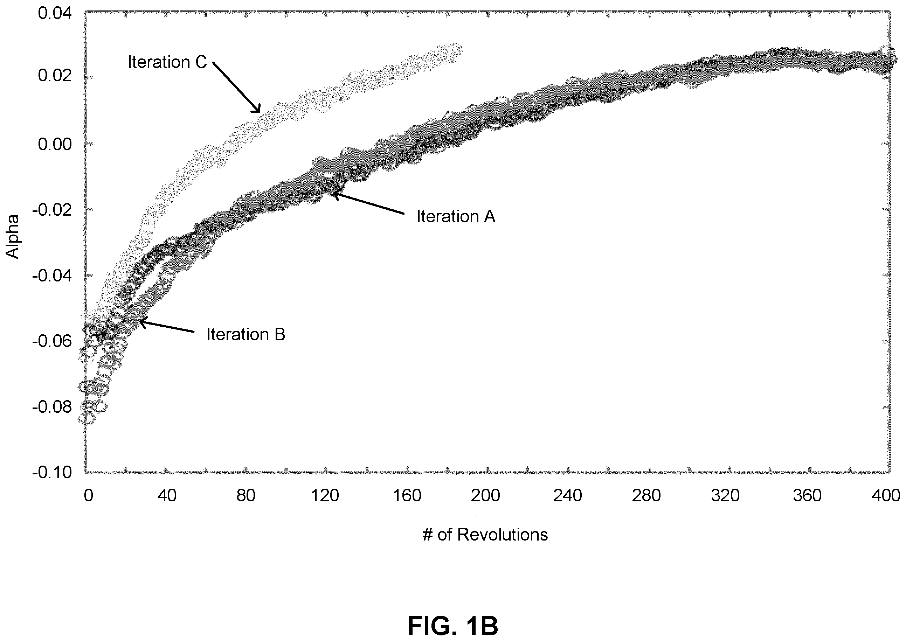

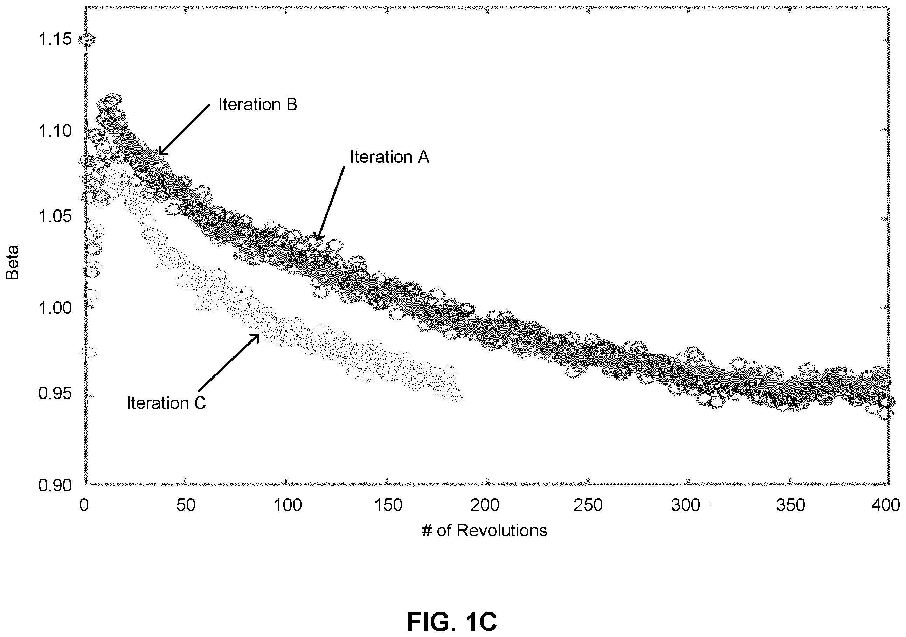

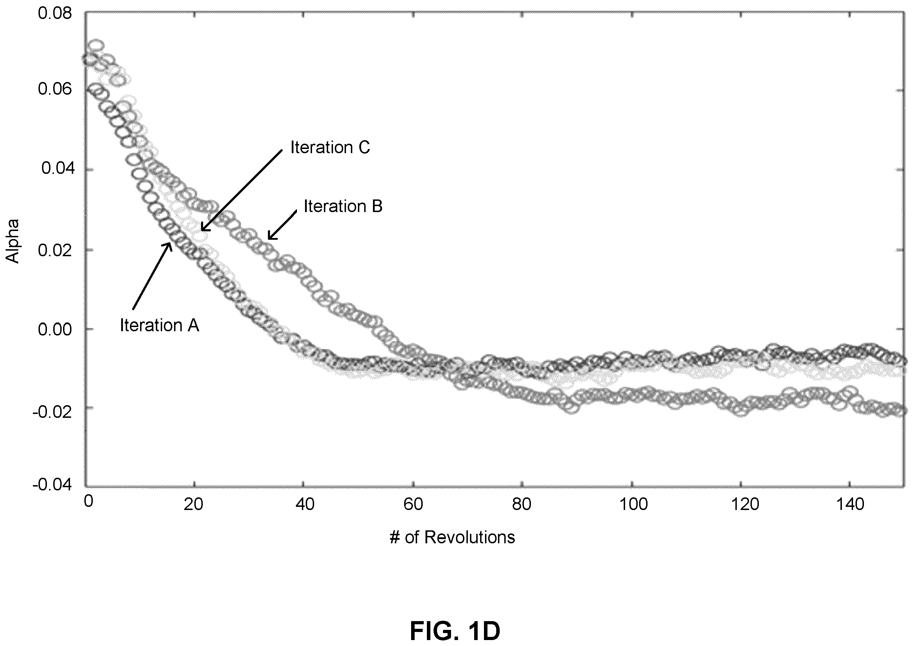

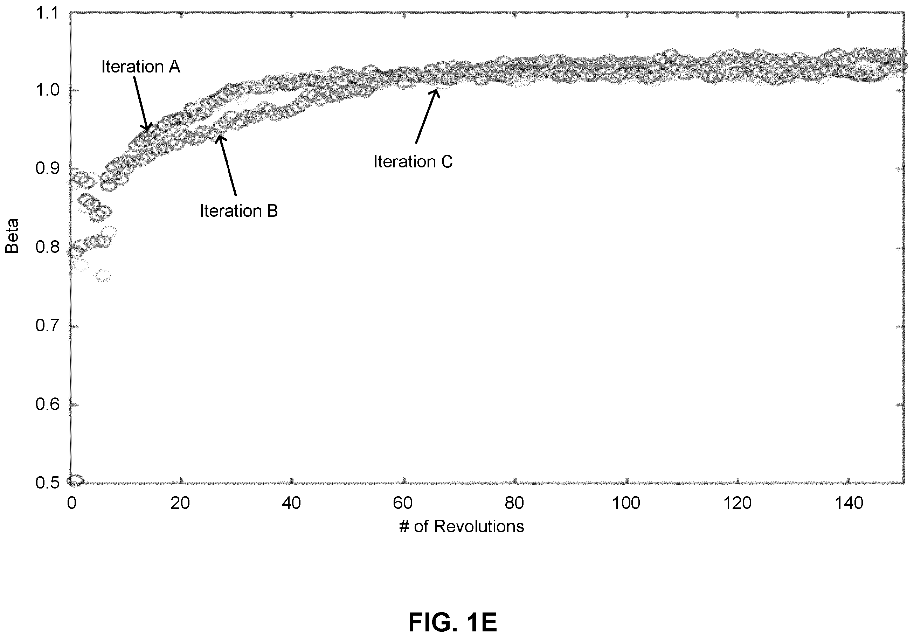

Figs. 1B-1E are diagrams illustrating examples of parameter profiles associated with three iterations of an example blending process. Figs. 1B and 1C are diagrams of $\alpha$ and $\beta$ profiles, respectively, for a first stage of blending for each of the three iterations (e.g., Iteration A, Iteration B, and Iteration C) of an example blending process, as extracted using an MSC model. Figs. 1D and 1E are diagrams of $\alpha$ and $\beta$ profiles, respectively, for a second stage of blending for each of the three iterations of the example blending process, as extracted using an MSC model.

Returning to Fig. 1A, as shown at reference 106, the detection device 220 may determine an end point of the iteration of the dynamic process based on the set of parameter profiles. In some implementations, the detection device 220 may determine the end point of the dynamic process using a shape-based analysis of the set of parameter profiles. In some implementations, the detection device 220 may perform the shape-based analysis using a set of slope thresholds associated with the iteration of the dynamic process. In some implementations, the detection device 220 may identify the set of slope thresholds based on a set of slope profiles associated with the set of parameter profiles and a set of trends associated with the set of parameter profiles.

Thus, in some implementations, the detection device 220 may generate a set of slope profiles based on the set of parameter profiles. Here, each slope profile in the set of slopes profiles may correspond to a respective parameter profile in the set of parameter profiles. Thus, in some implementations, the detection device 220 may generate a slope profile for each parameter profile in the set of parameter profiles. A slope profile is a profile representative of a slope of a parameter profile over a duration of the iteration of the dynamic process. In some implementations, to generate a slope profile, the detection device 220 computes the slope of a parameter profile along an entire length (e.g., in the time domain) of the parameter profile by fitting a low order polynomial regression model in a sliding window, and then differentiating the regression model.

In some implementations, the detection device 220 may perform normalization of the parameter profile prior to generating the slope profile. In some implementations, normalization of parameter profiles (i.e., parameter profiles from different iterations with different value ranges) makes the parameter profiles more directly comparable across different iterations of the dynamic process. The normalization technique used by the detection device 220 to normalize the parameter profile may be, for example, normalization by range, or may be another type of normalization technique.

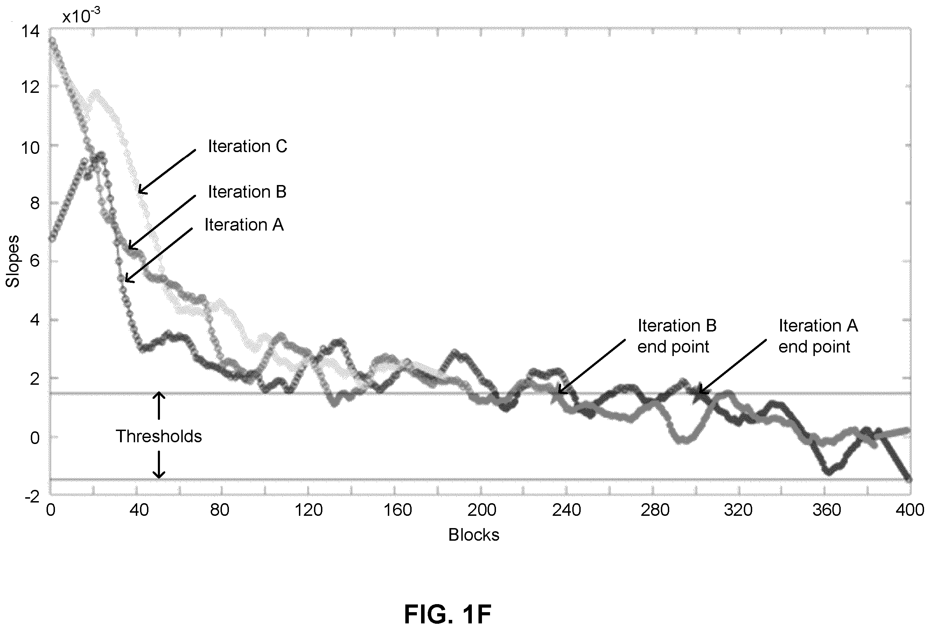

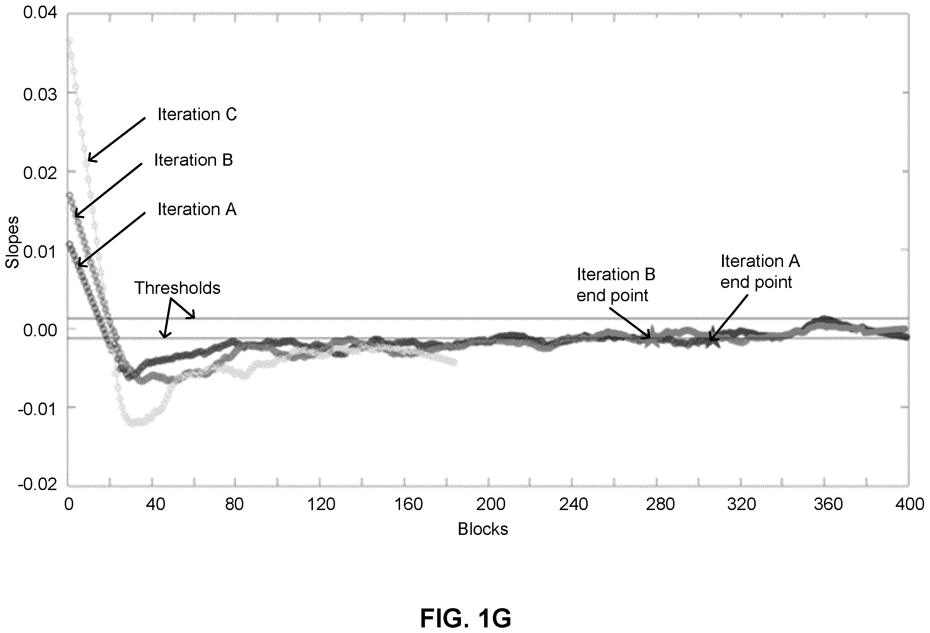

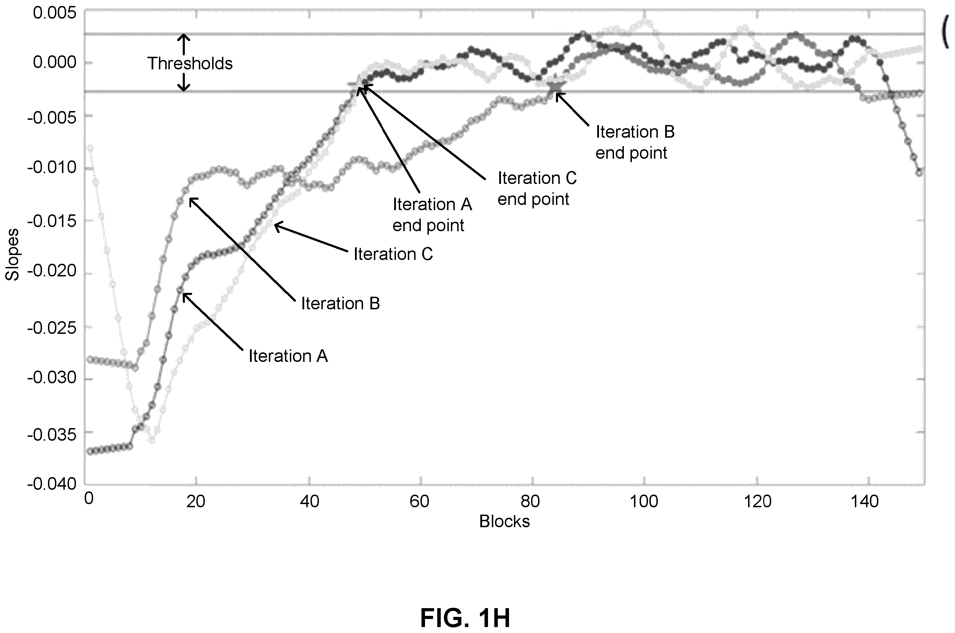

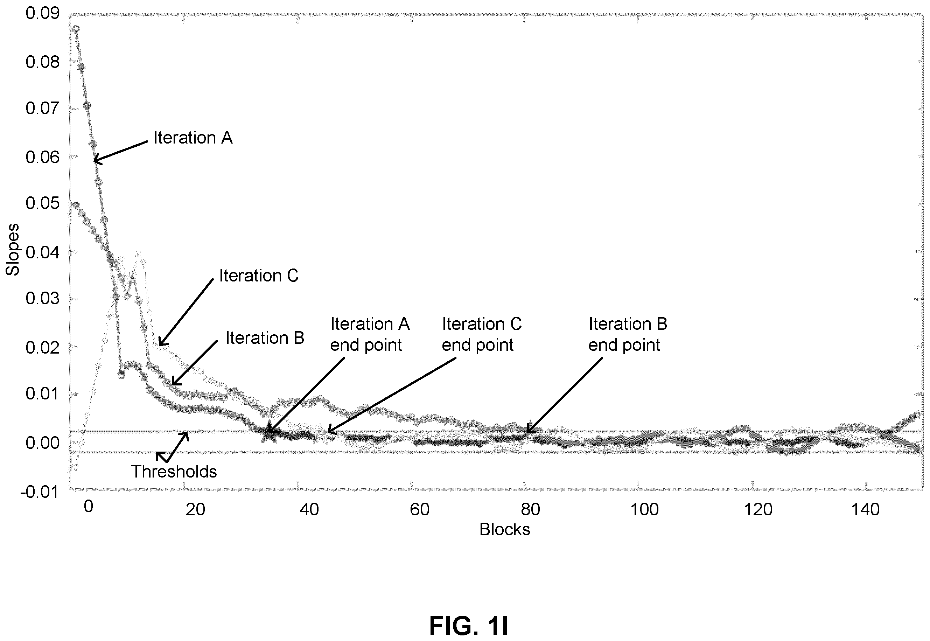

Figs. 1F-1I are diagrams illustrating examples of slope profiles corresponding to the parameter profiles associated with the three iterations of the example dynamic process as described above with respect to Figs. 1B-1E. Figs. 1F and 1G are diagrams of slope profiles of the $\alpha$ and $\beta$ profiles shown in Figs. 1B and 1C, respectively. Figs. 1H and 1I are diagrams of slope profiles of the $\alpha$ and $\beta$ profiles shown in Figs. 1D and 1E, respectively.

A plateau of a given parameter profile (i.e., when a slope of the given parameter profile is near zero) indicates that the iteration of the dynamic process has reached the steady state. Therefore, a slope of a parameter profile being near zero is indicative of the end point of the iteration of the dynamic process (i.e., that the iteration of the dynamic process has reached a steady state). Thus, a set of slope thresholds (e.g., an upper slope threshold and a lower slope threshold) needs to be selected, with the set of slope thresholds defining a range of values within which the slope is considered to be sufficiently close enough to zero for the purpose of determining an end point of the iteration of the dynamic process using a given slope profile. In some implementations, the detection device 220 may determine a set of slope thresholds for a given slope profile based on a trend of the parameter profile associated with the slope profile.

Thus, in some implementations, the detection device 220 may determine a trend of the parameter profiles in the set of parameter profiles. A trend of a parameter profile is an indication of whether the parameter profile trends upward or downward. In some implementations, the detection device 220 may determine the trend of the parameter profile based on a sign of the slope profile. As examples, the $\beta$ profile shown in Fig. 1C and the $\alpha$ profile shown in Fig. 1D trend downward (e.g., the parameter profiles have overall negative slopes), while the $\alpha$ profile shown in Fig. 1B and the $\beta$ profile shown in Fig. 1E trend upward (e.g., the parameter profiles have overall positive slopes).

In some implementations, the detection device 220 may identify the set of slope thresholds associated with a parameter profile based on the trend of the parameter profile and the slope profile associated with the parameter profile. In some implementations, if the trend of the parameter profile indicates that the parameter profile trends upward, then detection device 220 may determine the set of slope thresholds based on a minimum value of the slope profile. In some implementations, if the trend of the parameter profile indicates that the parameter profile trends downward, then the detection device 220 may determine the set of slope thresholds based on a maximum value of the slope profile. In some implementations, the detection device 220 may determine a set of slope thresholds associated with each parameter profile (e.g., the detection device 220 may determine a set of slope thresholds associated with the $\alpha$ parameter profile and a set of slope thresholds associated with the $\beta$ parameter profile).

Notably, spectral data from early stages of the iteration of the dynamic process may in some cases be highly unstable and, therefore, should not be used in association with determining an end point of the iteration of the dynamic process. Therefore, in some implementations, the detection device 220 may identify, based on the spectroscopic data, a starting time point of the spectroscopic data to be used for determining an end point of the iteration of the dynamic process. This time point may be referred to as a pseudo steady state. Thus, in some implementations, a starting time point associated with generating the parameter profile is at or after the identified starting time point of the spectroscopic data.

In some implementations, the detection device 220 may determine an end point of the iteration of the dynamic process based on the slope profile using the set of slope thresholds. For example, in some implementations, the detection device 220 may determine the end point based on a set of end point criteria. In one example, the set of end point criteria may require that a particular percentage of a quantity of consecutive values of the slope profile (e.g., 80% of a particular quantity of consecutive values of the slope profile) be within a lower slope threshold and an upper slope threshold. In some implementations, the detection device 220 may determine an end point of the iteration of the dynamic process for each parameter profile (e.g., the detection device 220 may determine an end point associated with the $\alpha$ parameter profile and an end point associated with the $\beta$ parameter profile). That is, the detection device 220 may in some aspects determine a set of candidate end points based on a set of slope profiles and associated sets of slope thresholds, where each candidate end point in the set of candidate end points is associated with a respective slope profile in the set of slope profiles. The detection device 220 may then determine the end point of the iteration of the dynamic process based on the set of candidate end points. For example, the detection device 220 may select the most conservative (e.g., latest in time) end point from among the set of candidate end points as the end point detected using the shape-based analysis.

In some implementations, the MSC-based end point determined by the detection device 220 can be compared to an end point determined based on a chemical signal associated with the same set of spectroscopic data. Here, the detection device 220 may be configured to select a final endpoint of the iteration of the blending process as the most conservative (e.g., latest in time) of the MSC-based end point or the end point detected based on the chemical signal. Thus, in some implementations, the detection device 220 may determine a first candidate end point of the iteration of the dynamic process based on a physical signal, and may determine a second candidate end point of the iteration of the dynamic process based on a chemical signal extracted from the spectroscopic data. The detection device 220 may then select either the first candidate end point or the second candidate end point as a final end point of the iteration of the dynamic process accordingly.

Additionally, or alternatively, the detection device 220 may determine the end point of the iteration of the dynamic process based on the parameter profiles (e.g., rather than based on slope profiles of the parameter profiles). In such an implementation, the detection device 220 may identify a set of parameter thresholds associated with the iteration of the dynamic process based on the set of parameter profiles, and may determine the end point of the iteration of the dynamic process based on the set of parameter profiles and the set of parameter thresholds. In some such implementations, an end point determined using a parameter profile itself can be considered a candidate end point in a pool of candidate end points, and the detection device 220 may select a final end point as described above (e.g., by selecting a most conservative end point from an MSC-based end point, an end point determined based on a chemical signal, and an end point determined based on a parameter profile itself).

In some implementations, the detection device 220 may perform end point detection for a single slope profile from a single iteration of the dynamic process. In such an implementation, the detection device 220 may use predefined end point criteria in association with determining the end point. In one example, the detection device 220 may determine a point in time at which a particular percentage (e.g., 80%) of a particular quantity of consecutive values (e.g., 30 consecutive values) of the slope profile are greater than or equal to the lower slope threshold and less than or equal to the upper slope threshold.

In some implementations, the detection device 220 may perform end point detection associated with multiple iterations of the dynamic process. For example, the above-described process can be performed for each iteration of the dynamic process separately, and a set of slope thresholds with a particular characteristic (e.g., a widest range) can be identified as a master set of slope thresholds for each parameter associated with the dynamic process. The detection device 220 may then use the master set(s) of slope thresholds to determine an end point of each iteration. For example, the master set(s) of slope thresholds can be used for determining an end point for a previously-performed iteration or for a later-performed iteration (e.g., for end point detection in real-time or near real-time).

Figs. 1F-1I show slope profiles, as noted above, along with MSC-based end points detected using the shape-based analysis described above. The end points shown in Figs. 1F-1I are summarized in Table 1:

Table 1

|  | Alpha Profiles | | Beta Profiles | |
|---|---|---|---|---|
|  | First Stage | Second Stage | First Stage | Second Stage |
| Iteration A | 302 | 49 | 307 | 35 |
| Iteration B | 236 | 84 | 278 | 81 |
| Iteration C | Not Detected | 49 | Not Detected | 44 |

As illustrated in Table 1, for the second stage of blending for Iteration A, the end point determined based on the $\beta$ profile (e.g., 35) was slightly earlier than the end point determined based on the $\alpha$ profile (e.g., 49). For the first stage of blending for Iteration B, the end point determined based on the $\beta$ profile (e.g., 278) was slightly later than the end point determined based on the $\alpha$ profile (e.g., 236). Here, the more conservative result (i.e., later-in-time end point), could be selected as the end point for these iterations.

For comparison, end points determined using two different other techniques (e.g., rolling principal component analysis (PCA) and MBM) that are based on chemical signals are summarized in Table 2:

Table 2

|  | Rolling PCA Profiles | | MBM Profiles | |
|---|---|---|---|---|
|  | First Stage | Second Stage | First Stage | Second Stage |
| Iteration A | 236 | 46 | 222 | 53 |
| Iteration B | 206 | 71 | 218 | 77 |
| Iteration C | Not Detected | 46 | Not Detected | 43 |

As illustrated in Table 2, the two conventional techniques show similar results with determined endpoints being slightly earlier than those determined using the MSC-based technique for the first stage of blending for Iteration A and Iteration B.

Returning to Fig. 1A, as shown at reference 108, the detection device 220 may (optionally) provide an indication of the end point of the iteration of the dynamic process. For example, the detection device 220 may provide, to a user device 230, an indication of the end point of the iteration of the dynamic process as determined by the detection device 220. In some implementations, the detection device 220 may provide information associated with the end point to the user device 230 to, for example, enable visualization of the slope thresholds during the later-performed iteration of the dynamic process.

Additionally, or alternatively, the detection device 220 may provide or store information associated with the set of slope thresholds. For example, the detection device 220 may store an indication of the set of slope thresholds as determined by the detection device 220 (e.g., such that the set of slope thresholds can be used for detection of an end point of another iteration of the dynamic process that is performed at a later time). As another example, the detection device 220 may provide an indication of the set of slope thresholds to a user device 230 (e.g., to enable visualization of the evolution of the dynamic process via the user device 230).

In this way, the detection device 220 may determine an end point of an iteration of a dynamic process based on a physical signal (e.g., changes of light scattering), thereby providing understanding of blend homogeneity from the physical perspective. In some implementations, such information can be complementary to information obtained based on chemical signals, thereby improving reliability of end point detection. Further, using the shape-based method described above, the detection device 220 can automatically determine MSC-based end points during post-analysis of one or more iterations of the dynamic process without a need for calibration or historical data or for a user to manually select end point detection thresholds. In some implementations, end point detection thresholds based on post-analysis of one or more iterations of the dynamic process can be used for monitoring future iterations of the dynamic process (e.g., in real-time or near real-time).

As indicated above, Figs. 1A-1I are provided as examples. Other examples are possible and may differ from what is described with regard to Figs. 1A-1I.

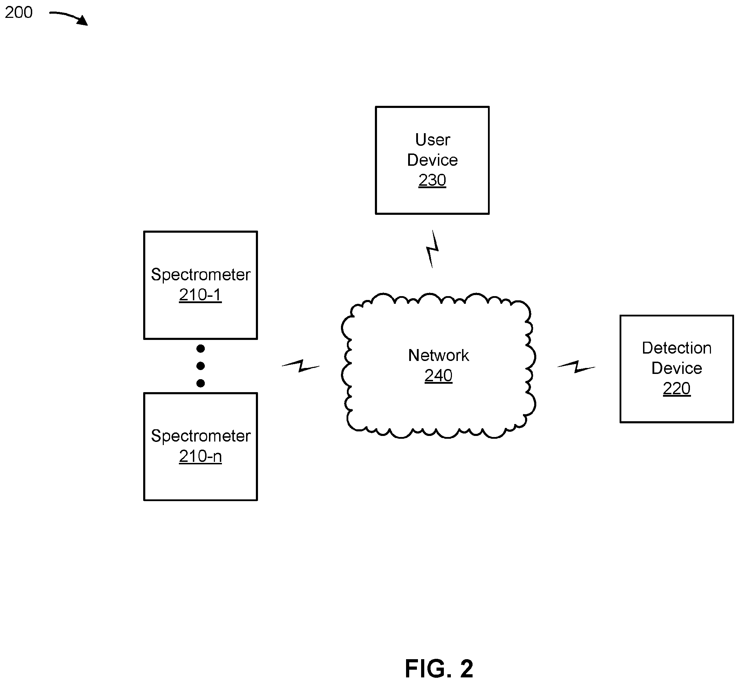

Fig. 2 is a diagram of an example environment 200 in which systems and/or methods described herein may be implemented. As shown in Fig. 2, environment 200 may include one or more spectrometers 210-1 through 210-n (n ≥ 1) (herein collectively referred to as spectrometers 210, and individually as spectrometer 210), a detection device 220, a user device 230, and a network 240. Devices of environment 200 may interconnect via wired connections, wireless connections, or a combination of wired and wireless connections.

Spectrometer 210 includes a device capable of performing a spectroscopic measurement on a sample (e.g., a sample associated with a manufacturing process). For example, spectrometer 210 may include a desktop (i.e., non-handheld) spectrometer device, a handheld spectrometer device, or a portable spectrometer device that performs spectroscopy (e.g., vibrational spectroscopy, such as NIR spectroscopy, mid-infrared spectroscopy (mid-IR), Raman spectroscopy, and/or the like). In some implementations, the spectrometer 210 may be installed or included in a device used in association with performing iterations of a dynamic process. For example, the spectrometer device 210 may in some implementations be installed or otherwise included in a blending device used to perform iterations of a blending process. In some implementations, spectrometer 210 may be capable of providing spectral data, obtained by spectrometer 210, for analysis by another device, such as detection device 220.

Detection device 220 includes one or more devices capable of performing one or more operations associated with determining an end point of an iteration of a dynamic process based on a set of parameter profiles, as described herein. For example, detection device 220 may include a server, a group of servers, a computer, a cloud computing device, and/or the like. In some implementations, detection device 220 may receive information from and/or transmit information to another device in environment 200, such as spectrometer 210 and/or user device 230.

User device 230 includes one or more devices capable of receiving, processing, and/or providing information associated with determining an end point of an iteration of a dynamic process based on a set of parameter profiles, as described herein. For example, user device 230 may include a communication and computing device, such as a desktop computer, a mobile phone (e.g., a smart phone, a radiotelephone, etc.), a laptop computer, a tablet computer, a handheld computer, a wearable communication device (e.g., a smart wristwatch, a pair of smart eyeglasses, etc.), or a similar type of device.

Network 240 includes one or more wired and/or wireless networks. For example, network 240 may include a cellular network (e.g., a 5G network, a 4G network, an long-term evolution (LTE) network, a 3G network, a code division multiple access (CDMA) network, etc.), a public land mobile network (PLMN), a local area network (LAN), a wide area network (WAN), a metropolitan area network (MAN), a telephone network (e.g., the Public Switched Telephone Network (PSTN)), a private network, an ad hoc network, an intranet, the Internet, a fiber optic-based network, a cloud computing network, and/or the like, and/or a combination of these or other types of networks.

The number and arrangement of devices and networks shown in Fig. 2 are provided as an example. In practice, there may be additional devices and/or networks, fewer devices and/or networks, different devices and/or networks, or differently arranged devices and/or networks than those shown in Fig. 2. Furthermore, two or more devices shown in Fig. 2 may be implemented within a single device, or a single device shown in Fig. 2 may be implemented as multiple, distributed devices. Additionally, or alternatively, a set of devices (e.g., one or more devices) of environment 200 may perform one or more functions described as being performed by another set of devices of environment 200.

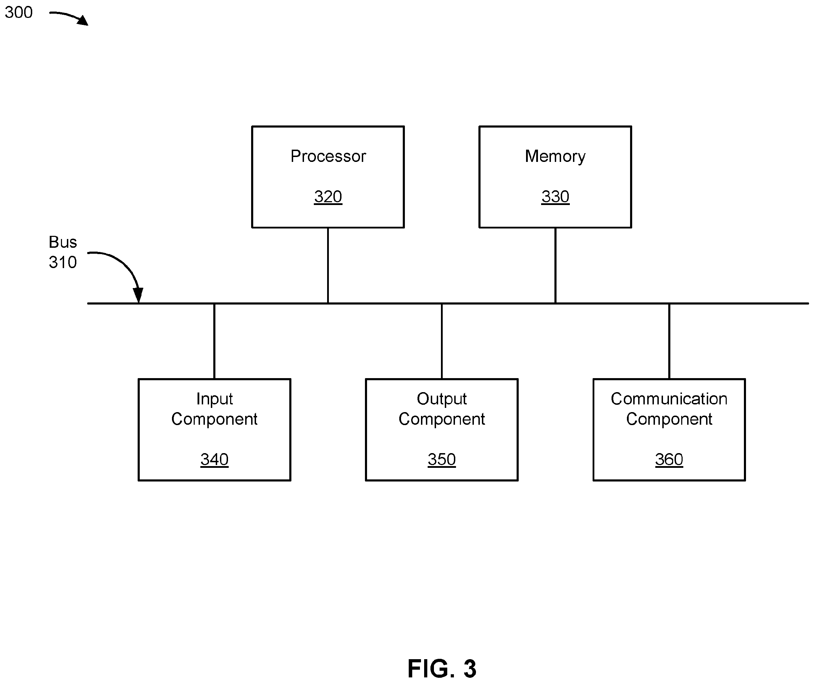

Fig. 3 is a diagram of example components of a device 300, which may correspond to spectrometer 210, detection device 220, and/or user device 230. In some implementations, spectrometer 210, detection device 220, and/or user device 230 may include one or more devices 300 and/or one or more components of device 300. As shown in Fig. 3, device 300 may include a bus 310, a processor 320, a memory 330, an input component 340, an output component 350, and a communication component 360.

Bus 310 includes one or more components that enable wired and/or wireless communication among the components of device 300. Bus 310 may couple together two or more components of Fig. 3, such as via operative coupling, communicative coupling, electronic coupling, and/or electric coupling. Processor 320 includes a central processing unit, a graphics processing unit, a microprocessor, a controller, a microcontroller, a digital signal processor, a field-programmable gate array, an application-specific integrated circuit, and/or another type of processing component. Processor 320 is implemented in hardware, firmware, or a combination of hardware and software. In some implementations, processor 320 includes one or more processors capable of being programmed to perform one or more operations or processes described elsewhere herein.

Memory 330 includes volatile and/or nonvolatile memory. For example, memory 330 may include random access memory (RAM), read only memory (ROM), a hard disk drive, and/or another type of memory (e.g., a flash memory, a magnetic memory, and/or an optical memory). Memory 330 may include internal memory (e.g., RAM, ROM, or a hard disk drive) and/or removable memory (e.g., removable via a universal serial bus connection). Memory 330 may be a non-transitory computer-readable medium. Memory 330 stores information, instructions, and/or software (e.g., one or more software applications) related to the operation of device 300. In some implementations, memory 330 includes one or more memories that are coupled to one or more processors (e.g., processor 320), such as via bus 310.

Input component 340 enables device 300 to receive input, such as user input and/or sensed input. For example, input component 340 may include a touch screen, a keyboard, a keypad, a mouse, a button, a microphone, a switch, a sensor, a global positioning system sensor, an accelerometer, a gyroscope, and/or an actuator. Output component 350 enables device 300 to provide output, such as via a display, a speaker, and/or a light-emitting diode. Communication component 360 enables device 300 to communicate with other devices via a wired connection and/or a wireless connection. For example, communication component 360 may include a receiver, a transmitter, a transceiver, a modem, a network interface card, and/or an antenna.

Device 300 may perform one or more operations or processes described herein. For example, a non-transitory computer-readable medium (e.g., memory 330) may store a set of instructions (e.g., one or more instructions or code) for execution by processor 320. Processor 320 may execute the set of instructions to perform one or more operations or processes described herein. In some implementations, execution of the set of instructions, by one or more processors 320, causes the one or more processors 320 and/or the device 300 to perform one or more operations or processes described herein. In some implementations, hardwired circuitry is used instead of or in combination with the instructions to perform one or more operations or processes described herein. Additionally, or alternatively, processor 320 may be configured to perform one or more operations or processes described herein. Thus, implementations described herein are not limited to any specific combination of hardware circuitry and software.

The number and arrangement of components shown in Fig. 3 are provided as an example. Device 300 may include additional components, fewer components, different components, or differently arranged components than those shown in Fig. 3. Additionally, or alternatively, a set of components (e.g., one or more components) of device 300 may perform one or more functions described as being performed by another set of components of device 300.

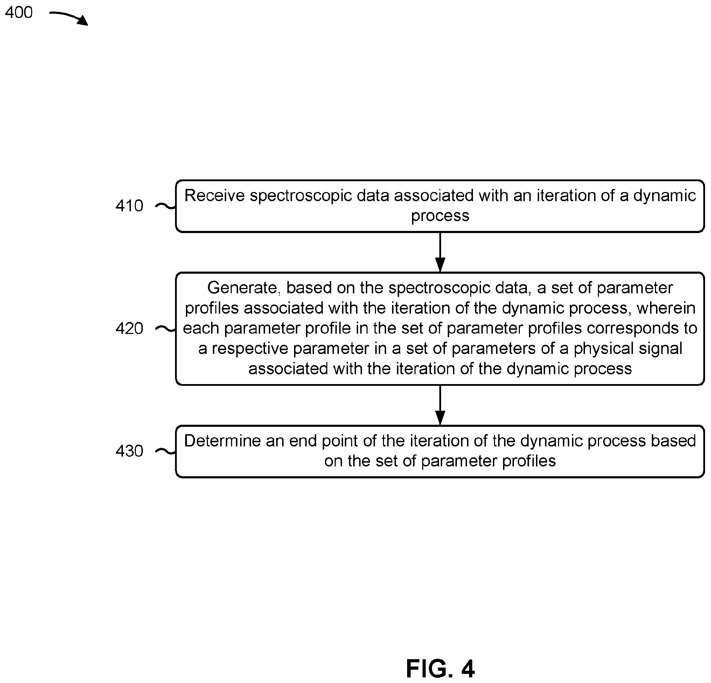

Fig. 4 is a flowchart of an example process 400 associated with determining an end point of an iteration of a dynamic process based on a set of parameter profiles. In some implementations, one or more process blocks of Fig. 4 are performed by a device (e.g., detection device 220). In some implementations, one or more process blocks of Fig. 4 are performed by another device or a group of devices separate from or including the device, such as a spectrometer (e.g., spectrometer 210) and/or a user device (e.g., user device 230). Additionally, or alternatively, one or more process blocks of Fig. 4 may be performed by one or more components of device 300, such as processor 320, memory 330, input component 340, output component 350, and/or communication component 360.

As shown in Fig. 4, process 400 may include receiving spectroscopic data associated with an iteration of a dynamic process (block 410). For example, the detection device may receive spectroscopic data associated with an iteration of a dynamic process, as described above.

As further shown in Fig. 4, process 400 may include generating, based on the spectroscopic data, a set of parameter profiles associated with the iteration of the dynamic process, wherein each parameter profile in the set of parameter profiles corresponds to a respective parameter in a set of parameters of a physical signal associated with the iteration of the dynamic process (block 420). For example, the detection device may generate, based on the spectroscopic data, a set of parameter profiles associated with the iteration of the dynamic process, wherein each parameter profile in the set of parameter profiles corresponds to a respective parameter in a set of parameters of a physical signal associated with the iteration of the dynamic process, as described above.

As further shown in Fig. 4, process 400 may include determining an end point of the iteration of the dynamic process based on the set of parameter profiles (block 430). For example, the detection device may determine an end point of the iteration of the dynamic process based on the set of parameter profiles, as described above.

Process 400 may include additional implementations, such as any single implementation or any combination of implementations described below and/or in connection with one or more other processes described elsewhere herein.

In a first implementation, the set of parameter profiles are generated using an MSC model that extracts the set of parameter profiles from the spectroscopic data.

In a second implementation, alone or in combination with the first implementation, the set of parameter profiles includes a profile of a parameter indicating an additive effect of light scattering during the iteration of the dynamic process.

In a third implementation, alone or in combination with one or more of the first and second implementations, the set of parameter profiles includes a profile of a parameter indicative of a multiplicative effect of light scattering during the iteration of the dynamic process.

In a fourth implementation, alone or in combination with one or more of the first through third implementations, determining the end point of the iteration of the dynamic process comprises determining a set of trends, wherein each trend in the set of trends corresponds to a respective parameter profile in the set of parameter profiles, generating, a set of slope profiles, wherein each slope profile in the set of slope profiles is associated with a respective parameter profile in the set of parameters profile, identifying sets of slope thresholds associated with the iteration of the dynamic process based on the set of trends and the set of slope profiles, determining a set of candidate end points based on the set of slope profiles and the sets of slope thresholds, wherein each candidate end point in the set of candidate end points is associated with a respective slope profile in the set of slope profiles, and determining the end point of the iteration of the dynamic process based on the set of candidate end points.

In a fifth implementation, alone or in combination with one or more of the first through fourth implementations, the end point is a first candidate end point, and the method further comprises determining a second candidate end point of the iteration of the dynamic process based on the spectroscopic data, wherein the second candidate end point is determined based on a chemical signal associated with the iteration of the dynamic process, and selecting either the first candidate end point or the second candidate end point as a final end point of the iteration of the dynamic process.

In a sixth implementation, alone or in combination with one or more of the first through fifth implementations, determining the end point of the iteration of the dynamic process comprises identifying a set of parameter thresholds associated with the iteration of the dynamic process based on the set of parameter profiles, and determining the end point of the iteration of the dynamic process based on the set of parameter profiles and the set of parameter thresholds.

In a seventh implementation, alone or in combination with one or more of the first through sixth implementations, process 400 includes identifying, based on the spectroscopic data, a starting time point of the spectroscopic data to be used for determining the end point of the iteration of the dynamic process, wherein a starting time point associated with generating the set of parameter profiles is at or after the identified starting time point of the spectroscopic data.

Although Fig. 4 shows example blocks of process 400, in some implementations, process 400 includes additional blocks, fewer blocks, different blocks, or differently arranged blocks than those depicted in Fig. 4. Additionally, or alternatively, two or more of the blocks of process 400 may be performed in parallel.

The foregoing disclosure provides illustration and description, but is not intended to be exhaustive or to limit the implementations to the precise forms disclosed. Modifications and variations may be made in light of the above disclosure or may be acquired from practice of the implementations.

As used herein, the term "component" is intended to be broadly construed as hardware, firmware, or a combination of hardware and software. It will be apparent that systems and/or methods described herein may be implemented in different forms of hardware, firmware, and/or a combination of hardware and software. The actual specialized control hardware or software code used to implement these systems and/or methods is not limiting of the implementations. Thus, the operation and behavior of the systems and/or methods are described herein without reference to specific software code - it being understood that software and hardware can be used to implement the systems and/or methods based on the description herein.

As used herein, satisfying a threshold may, depending on the context, refer to a value being greater than the threshold, greater than or equal to the threshold, less than the threshold, less than or equal to the threshold, equal to the threshold, not equal to the threshold, or the like.

Even though particular combinations of features are recited in the claims and/or disclosed in the specification, these combinations are not intended to limit the disclosure of various implementations. In fact, many of these features may be combined in ways not specifically recited in the claims and/or disclosed in the specification. Although each dependent claim listed below may directly depend on only one claim, the disclosure of various implementations includes each dependent claim in combination with every other claim in the claim set. As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiple of the same item.

When "a processor" or "one or more processors" (or another device or component, such as "a controller" or "one or more controllers") is described or claimed (within a single claim or across multiple claims) as performing multiple operations or being configured to perform multiple operations, this language is intended to broadly cover a variety of processor architectures and environments. For example, unless explicitly claimed otherwise (e.g., via the use of "first processor" and "second processor" or other language that differentiates processors in the claims), this language is intended to cover a single processor performing or being configured to perform all of the operations, a group of processors collectively performing or being configured to perform all of the operations, a first processor performing or being configured to perform a first operation and a second processor performing or being configured to perform a second operation, or any combination of processors performing or being configured to perform the operations. For example, when a claim has the form "one or more processors configured to: perform X; perform Y; and perform Z," that claim should be interpreted to mean "one or more processors configured to perform X; one or more (possibly different) processors configured to perform Y; and one or more (also possibly different) processors configured to perform Z."

No element, act, or instruction used herein should be construed as critical or essential unless explicitly described as such. Also, as used herein, the articles "a" and "an" are intended to include one or more items, and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, or a combination of related and unrelated items), and may be used interchangeably with "one or more." Where only one item is intended, the phrase "only one" or similar language is used. Also, as used herein, the terms "has," "have," "having," or the like are intended to be open-ended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of").

**Claims**

1. A method, comprising:

   receiving, by a device, spectroscopic data associated with an iteration of a dynamic process;
   generating, by the device and based on the spectroscopic data, a set of parameter profiles associated with the iteration of the dynamic process,
   wherein each parameter profile in the set of parameter profiles corresponds to a respective parameter in a set of parameters of a physical signal associated with the iteration of the dynamic process; and
   determining an end point of the iteration of the dynamic process based on the set of parameter profiles.

2. The method of claim 1, wherein the set of parameter profiles are generated using a multiplicative scatter correction (MSC) model that extracts the set of parameter profiles from the spectroscopic data.

3. The method of claim 1 or claim 2, wherein the set of parameter profiles includes a profile of a parameter indicating an additive effect of light scattering during the iteration of the dynamic process.

4. The method of any of claims 1 to 3, wherein the set of parameter profiles includes a profile of a parameter indicative of a multiplicative effect of light scattering during the iteration of the dynamic process.

5. The method of any of claims 1 to 4, wherein determining the end point of the iteration of the dynamic process comprises:

   determining a set of trends, wherein each trend in the set of trends corresponds to a respective parameter profile in the set of parameter profiles;
   generating, a set of slope profiles, wherein each slope profile in the set of slope profiles is associated with a respective parameter profile in the set of parameter profiles;
   identifying sets of slope thresholds associated with the iteration of the dynamic process based on the set of trends and the set of slope profiles;
   determining a set of candidate end points based on the set of slope profiles and the sets of slope thresholds, wherein each candidate end point in the set of candidate end points is associated with a respective slope profile in the set of slope profiles; and
   determining the end point of the iteration of the dynamic process based on the set of candidate end points.

6. The method of any of claims 1 to 5, wherein the end point is a first candidate end point, and the method further comprises:

determining a second candidate end point of the iteration of the dynamic process based on the spectroscopic data,
wherein the second candidate end point is determined based on a chemical signal associated with the iteration of the dynamic process; and
selecting either the first candidate end point or the second candidate end point as a final end point of the iteration of the dynamic process.

7. The method of any of claims 1 to 6, wherein determining the end point of the iteration of the dynamic process comprises:

identifying a set of parameter thresholds associated with the iteration of the dynamic process based on the set of parameter profiles; and
determining the end point of the iteration of the dynamic process based on the set of parameter profiles and the set of parameter thresholds.

8. The method of any of claims 1 to 7, further comprising identifying, based on the spectroscopic data, a starting time point of the spectroscopic data to be used for determining the end point of the iteration of the dynamic process, wherein a starting time point associated with generating the set of parameter profiles is at or after the identified starting time point of the spectroscopic data.

9. A device, comprising:

one or more memories; and
one or more processors, coupled to the one or more memories, configured to:

obtain spectroscopic data associated with an iteration of a dynamic process;
generate, based on the spectroscopic data, a set of parameter profiles associated with the iteration of the dynamic process,
wherein the set of parameter profiles are generated using a multiplicative scatter correction (MSC) model that extracts the set of parameter profiles from the spectroscopic data; and
determine an end point of the iteration of the dynamic process based on the set of parameter profiles.

10. The device of claim 9, wherein each parameter profile in the set of parameter profiles corresponds to a respective parameter in a set of parameters of a physical signal associated with the iteration of the dynamic process; and/or
wherein the set of parameter profiles includes a profile of a parameter indicating an additive effect of light scattering during the iteration of the dynamic process and/or wherein the set of parameter profiles includes a profile of a parameter indicative of a multiplicative effect of light scattering during the iteration of the dynamic process.

11. The device of claim 9 or claim 10:

wherein the one or more processors, to determine the end point of the iteration of the dynamic process, are configured to:

determine a set of trends, wherein each trend in the set of trends corresponds to a respective parameter profile in the set of parameter profiles;
generate, a set of slope profiles, wherein each slope profile in the set of slope profiles is associated with a respective parameter profile in the set of parameter profiles;
identify sets of slope thresholds associated with the iteration of the dynamic process based on the set of trends and the set of slope profiles;
determine a set of candidate end points based on the set of slope profiles and the sets of slope thresholds, wherein each candidate end point in the set of candidate end points is associated with a respective slope profile in the set of slope profiles; and
determine the end point of the iteration of the dynamic process based on the set of candidate end points; and/or

wherein the one or more processors, to determine the end point of the iteration of the dynamic process, are configured to:

identify a set of parameter thresholds associated with the iteration of the dynamic process based on the set of parameter profiles; and
determine the end point of the iteration of the dynamic process based on the set of parameter profiles and the set of parameter thresholds.

12. The device of any of claims 9 to 11:

wherein the end point is a first candidate end point, and the one or more processors are further configured to:

determine a second candidate end point of the iteration of the dynamic process based on the spectroscopic data,
wherein the second candidate end point is determined based on a chemical signal associated with the iteration of the dynamic process; and
select either the first candidate end point or the second candidate end point as a final end point of the iteration of the dynamic process; and/or

wherein the one or more processors are further configured to identify, based on the spectroscopic data, a starting time point of the spectroscopic data to be used for determining the end point of the iteration of the dynamic process, wherein a starting time point associated with generating the set of parameter profiles is at or after the identified starting time point of the spectroscopic data.

13. A non-transitory computer-readable medium storing a set of instructions, the set of instructions comprising:

one or more instructions that, when executed by one or more processors of a device, cause the device to:

receive spectroscopic data associated with an iteration of a dynamic process;
generate, based on the spectroscopic data, a set of parameter profiles associated with the iteration of the dynamic process, wherein the set of parameter profiles includes at least one of:

a profile of a parameter indicating an additive effect of light scattering during the iteration of the dynamic process, or
a profile of a parameter indicative of a multiplicative effect of light scattering during the iteration of the dynamic process; and

determine an end point of the iteration of the dynamic process based on the set of parameter profiles.

14. The non-transitory computer-readable medium of claim 13, wherein the set of parameter profiles are generated using a multiplicative scatter correction (MSC) model that extracts the set of parameter profiles from the spectroscopic data; and/or
wherein each parameter profile in the set of parameter profiles corresponds to a respective parameter in a set of parameters of a physical signal associated with the iteration of the dynamic process.

15. The non-transitory computer-readable medium of claim 13 or claim 14, wherein the one or more instructions, that cause the device to determine the end point of the iteration of the dynamic process, cause the device to:

determine a set of trends, wherein each trend in the set of trends corresponds to a respective parameter profile in the set of parameter profiles;
generate, a set of slope profiles, wherein each slope profile in the set of slope profiles is associated with a respective parameter profile in the set of parameter profiles;
identify sets of slope thresholds associated with the iteration of the dynamic process based on the set of trends and the set of slope profiles;
determine a set of candidate end points based on the set of slope profiles and the sets of slope thresholds, wherein each candidate end point in the set of candidate end points is associated with a respective slope profile in the set of slope profiles; and
determine the end point of the iteration of the dynamic process based on the set of candidate end points.

100 ⟶

**104**
Generate, based on spectroscopic data,
set of parameter profiles associated with
iteration of dynamic process

**106**
Determine end point of
iteration based on set of
parameter profiles

**108**
Indication of
end point of iteration

| Spectrometer(s) 210 | Detection device 220 | User device 230 |

102 ⟶ Spectroscopic data associated with iteration of dynamic process

**FIG. 1A**

EP 4 653 845 A1

FIG. 1B

FIG. 1C

FIG. 1D

**FIG. 1E**

FIG. 1F

EP 4 653 845 A1

**FIG. 1G**

**FIG. 1H**

**FIG. 1I**

200

Spectrometer
210-1

●
●
●

Spectrometer
210-n

User
Device
230

Network
240

Detection
Device
220

**FIG. 2**

300

EP 4 653 845 A1

Processor

320

Memory

330

Bus
310

Input
Component

340

Output
Component

350

Communication
Component

360

**FIG. 3**

400

410 — Receive spectroscopic data associated with an iteration of a dynamic process

420 — Generate, based on the spectroscopic data, a set of parameter profiles associated with the iteration of the dynamic process, wherein each parameter profile in the set of parameter profiles corresponds to a respective parameter in a set of parameters of a physical signal associated with the iteration of the dynamic process

430 — Determine an end point of the iteration of the dynamic process based on the set of parameter profiles

FIG. 4

EP 4 653 845 A1

## EUROPEAN SEARCH REPORT

**Application Number**

EP 25 17 4893

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/243699 A1 (SUN LAN [US] ET AL) 3 August 2023 (2023-08-03) * paragraphs [0003] - [0005], [0016] - [0031]; claims 1,2; figures 1A-1G, 3 * | 1-15 | INV. G01N21/359 B01F35/213 B01F35/22 |
| X | CN 107 843 574 B (UNIV BEIJING CHINESE MEDICINE; BEIJING TCMAGES PHARMACEUTICAL CO LTD) 29 May 2020 (2020-05-29) * claim 1; figures 1A-1D,2 * | 1-4,9,13 | ADD. G01N21/84 G01N21/65 |
| X | WU SI-JUN ET AL: "A near-infrared spectroscopy-based end-point determination method for the blending process of Dahuang soda tablets", JOURNAL OF ZHEJIANG UNIVERSITY-SCIENCE B, ZHEJIANG UNIVERSITY PRESS, HANGZHOU, vol. 21, no. 11, 1 November 2020 (2020-11-01), pages 897-910, XP037297091, ISSN: 1673-1581, DOI: 10.1631/JZUS.B2000417 [retrieved on 2020-11-17] * abstract; figures 1, 2, 6-8 * * page 901, right-hand column, last paragraph * | 1,9,13 | |
| X | JAUMOT J ET AL: "Blending process modeling and control by multivariate curve resolution", TALANTA, vol. 117, 27 September 2013 (2013-09-27), pages 492-504, XP028767809, ISSN: 0039-9140, DOI: 10.1016/J.TALANTA.2013.09.037 * abstract; figure 1 * * page 493, right-hand column, last paragraph - page 494, right-hand column, paragraph 2 * | 1,9,13 | |

**TECHNICAL FIELDS SEARCHED (IPC)**

B01F
G01N

The present search report has been drawn up for all claims

| Place of search | Date of completion of the search | Examiner |
|---|---|---|
| Munich | 2 October 2025 | Duijs, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 4893

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2025

| Patent document cited in search report | | Publication date | Patent family member(s) | | Publication date |
|---|---|---|---|---|---|
| US 2023243699 | A1 | 03-08-2023 | EP | 4220132 A1 | 02-08-2023 |
| | | | EP | 4556110 A2 | 21-05-2025 |
| | | | KR | 20230117505 A | 08-08-2023 |
| | | | US | 2023243699 A1 | 03-08-2023 |
| | | | US | 2024167872 A1 | 23-05-2024 |
| CN 107843574 | B | 29-05-2020 | NONE | | |

EPO FORM P0459

For more details about this annex : see Official Journal of the European Patent Office, No. 12/82